# EUROPEAN PATENT SPECIFICATION

(11) **EP 2 391 345 B1**
(45) Date of publication and mention of the grant of the patent: **03.09.2014**
(21) Application number: 10702092.7
(22) Date of filing: 27.01.2010
(51) Int. Cl.: A61K 9/14, A61K 9/20, A61K 9/28, A61K 9/48, A61K 9/00, A61K 31/165

(54) **Galenic formulations comprising aliskiren**
Galenische Formulierungen enthaltend Aliskiren
Formulations galéniques comprenant de l'aliskiren

(30) Priority: 28.01.2009 EP 09151496
(43) Date of publication of application: 07.12.2011
(73) Proprietor: Novartis AG, 4056 Basel (CH)
(72) Inventor: DESSET-BRÈTHES, Sabine, CH-4002 Basel (CH); HIRSCH, Stefan, CH-4002 Basel (CH)
(74) Representative: Schubert Santana, Isabelle
(86) International application number: PCT/EP2010/050886
(87) International publication number: WO 2010/086312

(56) References cited:
- WO-A1-2006/041974
- US-A1- 2004 266 743
- OH B H ET AL: "Aliskiren, an Oral Renin Inhibitor, Provides Dose-Dependent Efficacy and Sustained 24-Hour Blood Pressure Control in Patients With Hypertension" JOURNAL OF THE AMERICAN COLLEGE OF CARDIOLOGY, ELSEVIER, NEW YORK, NY, US, vol. 49, no. 11, 20 March 2007 (2007-03-20), pages 1157-1163, XP022666236 ISSN: 0735-1097 [retrieved on 2007-03-14]
- LOPES C M ET AL: "Compressed mini-tablets as a biphasic delivery system" INTERNATIONAL JOURNAL OF PHARMACEUTICS, ELSEVIER BV, NL, vol. 323, no. 1-2, 12 October 2006 (2006-10-12), pages 93-100, XP025113222 ISSN: 0378-5173 [retrieved on 2006-10-12]
- MUNDAY D L ET AL: "Controlled release delivery: Effect of coating composition on release characteristics of mini-tablets" INTERNATIONAL JOURNAL OF PHARMACEUTICS, ELSEVIER BV, NL, vol. 52, no. 2, 1 June 1989 (1989-06-01), pages 109-114, XP025544034 ISSN: 0378-5173 [retrieved on 1989-06-01]

## Description

The present invention relates to a solid oral dosage form as set out in claim 1 comprising an orally active renin inhibitor, Aliskiren, or a pharmaceutically acceptable salt thereof, particularly, a hemi-fumarate salt thereof, as the active ingredient in a suitable carrier medium and an outer coating in the form of a film-coat. In particular, the present invention provides a solid oral dosage form comprising Aliskiren, or a pharmaceutically acceptable salt thereof, particularly, a hemi-fumarate salt thereof, alone or in combination with another active agent, for use in the treatment of a disease or condition in a pediatric population and/or in patients which encounter problems with swallowing as a result of a disease or because of age.

In the following the term "Aliskiren", if not defined specifically, is to be understood both as the free base and as a salt thereof, especially a pharmaceutically acceptable salt thereof, particularly a hemi-fumarate thereof.

Renin released from the kidneys cleaves angiotensinogen in the circulation to form the decapeptide angiotensin I. This is in turn cleaved by angiotensin converting enzyme in the lungs, kidneys and other organs to form the octapeptide angiotensin II. The octapeptide increases blood pressure both directly by arterial vasoconstriction and indirectly by liberating from the adrenal glands the sodium-ion-retaining hormone aldosterone, accompanied by an increase in extracellular fluid volume. Inhibitors of the enzymatic activity of renin bring about a reduction in the formation of angiotensin I. As a result a smaller amount of angiotensin II is produced. The reduced concentration of that active peptide hormone is the direct cause of, e.g., the antihypertensive effect of renin inhibitors. Accordingly, renin inhibitors, or salts thereof, may be employed, e.g., as antihypertensives or for treating congestive heart failure.

The renin inhibitor, Aliskiren, in particular, a hemi-fumarate thereof, is known to be effective in the treatment of reducing blood pressure and is also well tolerated. Aliskiren in form of the free base is represented by the following formula 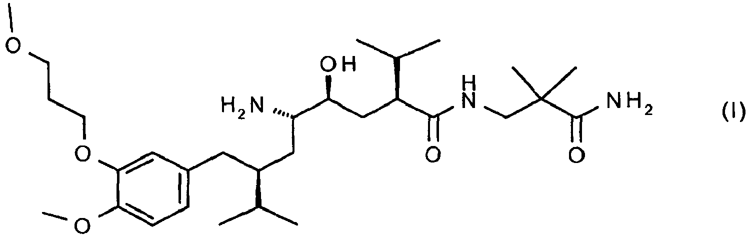 and chemically defined as 2(*S*),4(*S*),5(*S*),7(*S*)-*N*-(3-amino-2,2-dimethyl-3-oxopropyl)-2,7-di(1-methylethyl)-4-hydroxy-5-amino-8-[4-methoxy-3-(3-methoxy-propoxy)phenyl]-octanamide. As described above, most preferred is the hemi-fumarate salt thereof which is specifically disclosed in EP 678503 A as Example 83.

No studies in children with hypertension have been conducted with Aliskiren as mono-therapy or in combination with other antihypertensive agents to date. Since Aliskiren is the first of a new class of antihypertensive agents (Daugherty et al., 2008), it offers potential benefit compared to other current therapies available for children 0.5-17 years of age with hypertension.

As the prevalence of hypertension in children increases (Sorof, et al 2004), treatment alternatives to those currently available will be of increasing significance. Aliskiren may be particularly important in children 6-17 years of age with essential hypertension since Aliskiren has been shown to be of comparable efficacy in obese adult patients compared to those of normal body mass index (BMI) (Jensen, et al 2008). Obesity is thought to be a major contributor to essential hypertension in school age children and adolescents (Weinberger, et al 2008).

Hypertension in children, although less prevalent than hypertension in adults, represents an unmet medical need since pediatric patients with hypertension combined with renal dysfunction, cardiac disease or diabetes are more likely to require therapy with pharmacological agents. In addition, the increasing prevalence of obesity in school-age children and adolescents, worldwide, underlies the growing burden of pediatric hypertension (Weinberger, et al 2008). This is further complicated by the lack of pediatric dosing recommendations supported by controlled randomized clinical trials and age-appropriate pediatric drug formulations.

Currently available agents include ACE inhibitors, calcium channel blockers, β blockers and ARBs. A recent survey review of 438 North American pediatric nephrologists indicated that most used angiotensin-converting enzyme inhibitors (ACEIs), followed by calcium-channel blockers (47% and 37%, respectively), in the first-line treatment of primary hypertension. Beta -blockers were used as a first-line agent by only 7% of respondents, but in second-line therapy by 17% ( Chesney and Jones 2007). Diuretics, such as hydrochlorothiazide (HCTZ) have also been used in the treatment of hypertension in pediatric patients; however, no controlled pediatric hypertension studies have been done with HCTZ, thus far.

New antihypertensive treatment options such as Aliskiren may help physicians to address some of the issues cited above in adults. To date, there have been no studies with Aliskiren for the treatment of hypertension in children; a drug which offers a different mechanism of action and thus would have the potential to provide a treatment alternative for hypertensive children 0.5-17 years of age.

In pediatric use, oral administration of pharmaceutical agents in form of solutions, powders granules or tablets or film-coated tablets or capsules has certain advantages over alternative administration forms such as parenteral administration, i.e. i.v. or i.m. administration. Diseases requiring treatment with painful injectable formulations are considered to be more serious than those conditions which can be treated with oral dosage forms. However, the major advantage with oral formulations is held to be their suitability for self administration whereas parenteral formulations have to be administered in most cases by a physician or paramedical personnel.

The marketed Aliskiren is available in an oral dosage form as an immediate release film-coated tablet down to strength of 150mg. This is a non-divisible, round tablet with approx. 11mm diameter and approx. 4mm thickness and thus does not fulfill the requirements of a pediatric formulation in terms of strength, dosing flexibility, patient acceptability (swallow ability) and size.

Also the physical-chemical properties and the extremely bitter taste of the drug substance precludes the development of a liquid or oral formulation which is standard of care for children 0.5-17 years of age.

It is, therefore, an unmet need to develop and provide a treatment regime for treating hypertension in children and further a pediatric formulation of Aliskiren that does away with the deficiencies of the marketed Aliskiren immediate release film-coated tablet in terms of a pediatric application, that is a formulation that has the size, dosing flexibility and taste required for an application in children and maintains a bioavailability comparable to the marketed drug product.

However, most of the commonly used pediatric formulations which are appropriate for children, such as syrups and other liquid formulations, are not called for due to the physical-chemical properties and the extremely bitter taste of Aliskiren, which prohibit the development of such commonly used pediatric formulations. It was found, for example, that it was not feasible to develop liquid formulations of Aliskiren, because no sweeteners and/or flavors and/or viscosity modifiers could be identified that provided a sufficient taste masking to achieve a taste that is acceptable for children. The development of a liquid formulation, e.g. in form of a suspension with less bitter taste, was further prevented due to the very high water solubility (>350g/l between pH 1 and 8) of the Aliskiren compound. Furthermore, a previous clinical study had shown that the bioavailability of liquid Aliskiren formulation was lower than that of the solid oral forms.

Also coated powder or coated granules for reconstitution were not considered suitable since the expected high amounts of polymer required to achieve taste-masking with the high surface area would not have been acceptable for pediatric use in the proposed age groups. Further, the physical-chemical properties of Aliskiren also do not favor coated powder or coated granules due to the high hygroscopic nature of the compound.
This high hygroscopicity of Aliskiren would argue against the development of a drug product/formulation with such a high surface area because of the moisture uptake and its impact on stability, which was already shown to be a problem for the marketed Aliskiren film coated tablet formulations bound for adult population.
Further, film-coated pellets layered with Aliskiren were also not considered to be suitable as a dosage form, because the drug substance changed its polymorphic behavior to amorphous which may have detrimental effect on drug product stability.

Also the preparation of oral formulations in form of tablets in a reliable and robust way was known to be technically difficult.

One of these difficulties is caused, for example, by the needle shaped crystal habit of Aliskiren, which has a negative influence on the bulk properties of the drug substance, e.g., flow properties and bulk density. The compression behavior of the drug substance is poor, leading to weak interparticulate bonds and polymorphism changes under pressure. Aliskiren has a strong elastic component that also leads to weakening of interparticulate bonds. The drug substance quality is variable with effect on the processability of a tablet, e.g., particle size distribution, bulk density, flowability, wetting behavior, surface area and sticking tendency.

Moreover, Aliskiren is highly hygroscopic. In contact with water, the drug substance polymorphism changes to an amorphous state, which shows inferior stability compared to the crystalline state. The combination of these hurdles makes a standard tablet manufacturing process extremely difficult.

Direct compression is not a feasible option for routine production of Aliskiren because of, e.g., the needle shaped particle structure, the poor flowability with resulting processability problems and dose uniformity problems.

Another dosage form sometimes considered for pediatric use are formulations in form of minitablets, which may be provided with or without small amount of soft food, e.g. pudding or mashed vegetables. However, the combination of the above summarized technical difficulties makes a standard tablet manufacturing process for the production of minitablets such as that described in WO2005/089729 extremely difficult. Due to these technical difficulties this option was also not expected to lead to an acceptable result.

Irrespective of the technical difficulties in the formulation of Aliskiren, suitable process adaptations were developed within the scope of the present invention in the production of minitablets, which made it possible to overcome the many technical difficulties and to provide a solid unit dosage form of Aliskiren for oral administration in form of a minitablet having a core and an outer coating, wherein:
- the core comprises a therapeutically effective amount of Aliskiren, or a pharmaceutically acceptable salt thereof, and
- the outer coating is in form of a film-coat which comprises a taste masking material selected from polyacrylates, and/or a modified release coating component selected from cellulose derivatives an acrylic copolymers, and mixtures thereof,
wherein said minitablet has a size of between 1 mm and 4 mm, and wherein said minitablet contains between about 2 mg and about 4 mg of Aliskiren.

In particular, the present invention provides a solid unit dosage form of Aliskiren for oral administration in form of coated minitablets as an age-appropriate pediatric formulation, meeting the technical, administration and pharmacokinetic requirements.

The unit dosage form according to the present invention is not restricted to an application in children but can generally be used, for example, in patients with difficulties in swallowing due to a disease or age of the patient.

In one embodiment of the invention, a solid unit dosage form as described herein above is provided, wherein said minitablet has a size of between 1 mm and 3 mm, particularly of between 1.25 mm and 2.5 mm, but particularly of between 1.5 mm and 2.5 mm.

In one embodiment of the invention, the minitablet has a size of 2 mm.

In one embodiment of the invention, a solid unit dosage form in form of a minitablet as described herein above is provided, wherein said minitablet contains the active agent consisting entirely of Aliskiren, or a pharmaceutically acceptable salt thereof in an amount ranging from about between 2 mg to about 4 mg of the free base per unit dosage form, particularly from about between 2.5 mg and 3.5 mg of the free base per unit dosage form.

In one embodiment of the invention, the minitablet contains 3.125 mg Aliskiren or a pharmaceutically acceptable salt thereof/tablet.

In one embodiment of the invention, a solid unit dosage form as described herein above is provided, wherein said film-coat has a controlled release functionality.

In one embodiment of the invention, said film-coat has a pH-dependent release profile.

In one embodiment, a solid unit dosage form according to the invention and as described herein is provided, wherein said film-coat leads to an *in vitro* dissolution of Aliskiren of about 75% or less after 10 minutes, of about 96% or less after 20 minutes and of about 98% or less after 30 minutes, at a pH of about 2 (variant A).

In one embodiment, a solid unit dosage form according to the invention and as described herein is provided, wherein said film-coat leads to an *in vitro* dissolution of Aliskiren of about 70% or less after 10 minutes, of about 95% or less after 20 minutes and of about 98% or less after 30 minutes, at a pH of about 4.5 (variant A).

In one embodiment, a solid unit dosage form according to the invention and as described herein is provided, wherein said film-coat leads to an *in vitro* dissolution of Aliskiren of about 4% or less after 10 minutes, of about 32% or less after 20 minutes and of about 70% or less after 30 minutes, at a pH of about 6.8 (variant A).

In one embodiment, a solid unit dosage form according to the invention and as described herein is provided, wherein said film-coat comprises a basic butylated methacrylate copolymer as the film forming agent.

In one embodiment , a solid unit dosage form according to the invention and as described herein is provided, wherein said film-coat comprises an ethylcellulose aqueous dispersion as the film forming agent and, optionally, a hypromellose as a pore forming agent in the film forming agent.

In one embodiment, a solid unit dosage form according to the invention and as described herein is provided, wherein said film-coat comprises an ammonium methacrylate copolymer, particularly an ammonium methacrylate copolymer type A and/or an ammonium methacrylate copolymer Type B as the film forming agent.

In one embodiment, a solid unit dosage form as set out in the claims is provided, for oral administration comprising a therapeutically effective amount of Aliskiren, or a pharmaceutically acceptable salt thereof, particularly, a hemi-fumarate salt thereof, for use in the treatment of a disease or condition in a pediatric population.

In one embodiment, a solid unit dosage form as set out in the claims is provided, for use in the treatment of hypertension, congestive heart failure, angina, myocardial infarction, atherosclerosis, diabetic nephropathy, diabetic cardiac myopathy, renal insufficiency, peripheral vascular disease, left ventricular hypertrophy, cognitive dysfunction, stroke, headache and chronic heart failure.

### Definitions:

The terms "effective amount" or "therapeutically effective amount" refers to the amount of the active ingredient or agent which halts or reduces the progress of the condition being treated or which otherwise completely or partly cures or acts palliatively on the condition.

The term "Aliskiren," refers to a compound represented by the following formula 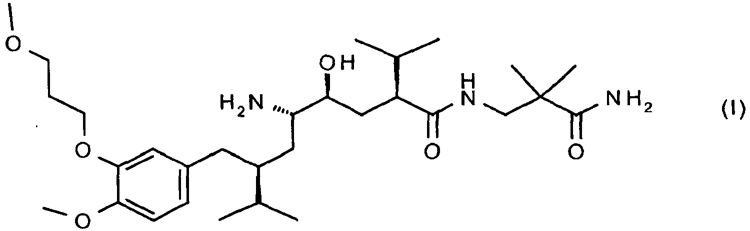 and chemically defined as 2(*S*),4(*S*),5(*S*),7(*S*)-*N*-(3-amino-2,2-dimethyl-3-oxopropyl)-2,7-di(1-methylethyl)-4-hydroxy-5-amino-8-[4-methoxy-3-(3-methoxy-propoxy)phenyl]-octanamide.

In the above and in the following the term "Aliskiren," if not defined specifically, is to be understood both as the free base and as a salt thereof, especially a pharmaceutically acceptable salt thereof, such as a hemi-fumarate, hydrogen sulfate, orotate or nitrate, most preferably a hemi-fumarate thereof.
As described above, most preferred is the hemi-fumarate salt thereof which is specifically disclosed in EP 678503 A as Example 83.

Aliskiren, in particular, a hemi-fumarate thereof, is a renin inhibitor known to be effective in the treatment of reducing blood pressure irrespective of age, sex or race and is also well tolerated.

The term "release" as used herein refers to a process by which the pharmaceutical oral dosage form is brought into contact with a fluid and the fluid transports the drug(s) outside the dosage form into the fluid that surrounds the dosage form. The combination of delivery rate and delivery duration exhibited by a given dosage form in a patient can be described as its in vivo release profile. The release profiles of dosage forms may exhibit different rates and durations of release and may be continuous. Continuous release profiles include release profiles in which one or more active ingredients are released continuously, either at a constant or variable rate.

For the purposes of the present application, an immediate release formulation is a formulation showing a release of the active substance(s), which is not deliberately modified by a special formulation design or manufacturing method.

For the purposes of the present application, a "modified release formulation" is a formulation showing a release of the active substance(s), which is deliberately modified by a special formulation design or manufacturing method. This modified release can be typically obtained by delaying the time of release of the active ingredient. Typically for the purposes of the present invention, a modified release refers to a release delayed by 30-60 mins.

The term "time delay" as used herein refers to the period of time between the administration of a dosage form comprising the composition of the invention and the release of the active ingredient from a particular component thereof.

The term "lag time" as used herein refers to the time between the release of the active ingredient from one component of the dosage form and the release of the active ingredient from another component of the dosage form.

The term "disintegration" as used herein refers to a process where the pharmaceutical oral dosage form, typically by means of a fluid, falls apart into separate particles and is dispersed. Disintegration is achieved when the solid oral dosage form is in a state in which any residue of the solid oral dosage form, except fragments of insoluble coating or capsule shell, if present, remaining on the screen of the test apparatus is a soft mass having no palpably firm core in accordance with USP<701>. The fluid for determining the disintegration property is water, such as tap water or deionized water. The disintegration time is measured by standard methods known to the person skilled in the art, see the harmonized procedure set forth in the pharmacopeias USP <701> and EP 2.9.1 and JP.

The term "dissolution" as used herein refers to a process by which a solid substance, here the active ingredient, is dispersed in molecular form in a medium. The dissolution rate of the active ingredient of the pharmaceutical oral dosage form of the invention is defined by the amount of drug substance that goes in solution per unit time under standardized conditions of liquid/solid interface, temperature and solvent composition. The dissolution rate is measured by standard methods known to the person skilled in the art, see the harmonized procedure set forth in the pharmacopeias USP <711> and EP 2.9.3 and JP. For the purposes of this invention, the test is for measuring the dissolution of the individual active ingredient is performed following pharmacopeia USP <711> at pH 2.0 using a basket method at 100 rpm (rotations per minute). The dissolution medium is preferably a buffer, typically a phosphate buffer, especially one as described in the example "Dissolution Test". The molarity of the buffer is preferably 0.1 M.

The term "minitablets" within the scope of this application denotes small tablets with an overall weight of approximately 2 to 30 mg, e.g. approximately 4 to 9 mg, e.g. approximately 7 mg, in their uncoated form. Minitablets are a specific form of multiparticulates as defined herein. They can be prepared as described herein, including preparation from other, smaller multiparticulates, such as particles, granules or beads. The minitablets may have any shape known to the skilled person for tablets, e.g. round e.g. with a diameter of about 1.25 to 3 mm; cylindrical e.g. having a convex upper face and convex lower face and e.g. with a cylindrical diameter and height independently of each other are from 1 to 3 mm; or biconvex minitablets e.g. whose height and diameter are approximately equal and are from 1.25 to 3 mm.

The core of the minitablet according to the invention may comprise additives or excipients that are suitable for the preparation of the solid oral dosage form according to the present invention. Tabletting aids, commonly used in tablet formulation can be used and reference is made to the extensive literature on the subject, see in particular Fiedler's "Lexikon der Hilfstoffe," 4th Edition, ECV Aulendorf 1996, which is incorporated herein by reference. These include, but are not limited to, fillers, binders, disintegrants, lubricants, glidants, stabilizing agents, fillers or diluents, surfactants, film-formers, softeners, pigments and the like.

The present invention likewise relates to a solid oral dosage form comprising a therapeutically effective amount of Aliskiren, or a pharmaceutically acceptable salt thereof, as an active agent, and a filler as an additive. Further additives include, but are not limited to, binders, disintegrants, lubricants, glidants, stabilizing agents, diluents, surfactants, film formers, pigments, softeners and antitacking agents and the like. The amounts of the active ingredient and further additives are preferably those as defined above.

The present invention likewise relates to a solid oral dosage form comprising a therapeutically effective amount of Aliskiren, or a pharmaceutically acceptable salt thereof, as an active agent, and a filler and a disintegrant as additives. Further additives include, but are not limited to, binders, lubricants, glidants, stabilizing agents, diluents, surfactants, film formers, pigments, softeners and antitacking agents and the like. The amounts of the active ingredient and further additives are preferably those as defined herein above.

The present invention likewise relates to a solid oral dosage form comprising a therapeutically effective amount of Aliskiren, or a pharmaceutically acceptable salt thereof, as an active agent, and a filler, a disintegrant and a lubricant as additives. Further additives include, but are not limited to, binders, glidants, stabilizing agents, diluents, surfactants, film formers, pigments, softeners and antitacking agents and the like. The amounts of the active ingredient and further additives are preferably those as defined herein above.

The present invention likewise relates to a solid oral dosage form comprising a therapeutically effective amount of Aliskiren, or a pharmaceutically acceptable salt thereof, as an active agent, and a filler, a disintegrant, a lubricant and a glidant as additives Further additives include, but are not limited to, binders, stabilizing agents, diluents, surfactants, film formers, pigments, softeners and antitacking agents and the like. The amounts of the active ingredient and further additives are preferably those as defined herein above.

The present invention likewise relates to a solid oral dosage form comprising a therapeutically effective amount of Aliskiren, or a pharmaceutically acceptable salt thereof, as an active agent, and a filler, a disintegrant, a lubricant, a glidant and a binder as additives Further additives include, but are not limited to, stabilizing agents, diluents, surfactants, film formers, pigments, softeners and antitacking agents and the like. The amounts of the active ingredient and further additives are preferably those as defined herein above.

One or more of these additives can be selected and used by a person skilled in the art having regard to the particular desired properties of the solid oral dosage form by routine experimentation and without any undue burden.

In one embodiment the solid oral dosage form according to the present invention comprises as an additive a filler or a diluent such as, for example, confectioner's sugar, compressible sugar, dextrates, dextrin, dextrose, lactose, mannitol, starches, e.g., potato starch, wheat starch, corn starch, hydroxypropyl cellulose, hydroxyethyl cellulose, hydroxypropyl methyl cellulose (HPMC), powdered cellulose, sorbitol, sucrose and talc. preferably, microcrystalline cellulose, e.g., products available under the registered trade marks AVICEL, FILTRAK, HEWETEN or PHARMACEL. A most preferred filler is microcrystalline cellulose, in particular, having a density of about 0.45g/cm³, e.g., AVICEL.

As disintegrants one can particularly mention carboxymethylcellulose calcium (CMC-Ca), carboxymethylcellulose sodium (CMC-Na), crosslinked PVP (e.g. CROSPOVIDONE, POLYPLASDONE or KOLLIDON XL), alginic acid, sodium alginate and guar gum, most preferably crosslinked PVP (CROSPOVIDONE), crosslinked CMC (Ac-Di-Sol), carboxymethylstarch-Na (PRIMOJEL and EXPLOTAB). A most preferred disintegrant is CROSPOVIDONE.

As lubricants one can mention in particular Mg stearate, aluminum (Al) or Ca stearate, PEG 4000 to 8000 and talc, hydrogenated castor oil, stearic acid and salts thereof, glycerol esters, Na-stearylfumarate, hydrogenated cotton seed oil and others. A most preferred lubricant is Mg stearate.

As glidants one can mention in particular colloidal silica, such as colloidal silicon dioxide, e.g., AEROSIL, magnesium (Mg) trisilicate, powdered cellulose, starch, talc and tribasic calcium phosphate or combinations of these with fillers or binders, e.g., silicified microcrystalline cellulose (PROSOLV). A most preferred glidant is colloidal silicon dioxide (e.g. AEROSIL 200).

As binders for wet granulation, one can particularly mention polyvinylpyrrolidones (PVP), e.g., PVP K 30, HPMC, e.g., viscosity grades 3 or 6 cps, and polyethylene glycols (PEG), e.g., PEG 4000. A most preferred binder is PVP K 30.

Hence, in one embodiment the solid oral dosage form according to the present invention comprises as an additive a filler, particularly microcrystalline cellulose.

In one embodiment the solid oral dosage form according to the present invention comprises as an additive, in addition to a filler, a disintegrant, particularly microcrystalline cellulose and Crospovidone.

In one embodiment the solid oral dosage form according to the present invention comprises as an additive, in addition to a filler and a disintegrant, a lubricant, particularly microcrystalline cellulose, Crospovidone and magnesium stearate.

In one embodiment the solid oral dosage form according to the present invention comprises as an additive, in addition to a filler, a disintegrant and a lubricant, a glidant, particularly microcrystalline cellulose, Crospovidone, magnesium stearate and colloidal silicon dioxide.

In one embodiment the solid oral dosage form according to the present invention comprises as an additive, in addition to a filler, a disintegrant, a lubricant and a glidant, a binder, particularly microcrystalline cellulose, Crospovidone, magnesium stearate, colloidal silicon dioxide and Povidone.

In particular, the cores of the minitablet according to the present invention and as described herein may be compressed from a mixture of the same granulate as the SPP100 marketed formulation of Aliskiren and an outer or external phase, particularly an external phase comprising a higher amount of magnesium stearate and no superdisintegrant compared to the reference marketed formulation.

The amount of each type of additive employed, e.g., glidant, binder, disintegrant, filler or diluent and lubricant or film coat may vary within ranges conventional in the art. Thus, for example, the amount of lubricant may vary within a range of from 0.2 to 5% by weight, in particular, for Mg stearate from 1.0% to 3.0% by weight, e.g., from 1.5% to 2.5% by weight; the amount of binder may vary within a range of from 0 to about 20% by weight, e.g., from 2.5% to 4.5% by weight; the amount of disintegrant may vary within a range of from 0 to about 20% by weight, e.g., from 3.% to 5% by weight; the amount of filler or diluent may vary within a range of from 0 to about 80% by weight, e.g., from 20 to 50% by weight; whereas the amount of glidant may vary within a range of from 0 to about 5% by weight, e.g. from 0.4 to 1.0% by weight; and the amount of film coat may vary within a range of 0 to 5 mg/cm², e.g. 0.4 mg/cm² to 0.7 mg/cm².

It is a characteristic of the present solid oral dosage forms that they contain only a relatively small amount of additives given the high content of the active agent. This enables the production of physically small unit dosage forms. The total amount of additives in a given uncoated unit dosage may be about 65% or less by weight based on the total weight of the solid oral dosage form, more particularly about 60% or less. Preferably, the additive content is in the range of about 35% to 58% by weight, more particularly, the additive content ranges from about 50% to about 56% by weight based on the hemifumarate.

A preferred amount of a filler in the tablet core, especially of microcrystalline cellulose, ranges from about 30% to 40%, particularly from about 33% to 40%, by weight per unit dosage form.

A preferred amount of a binder in the tablet core especially of Povidone, ranges from about 2.5% to 4.5%, particularly from about 3% to 4%, by weight per unit dosage form.

A preferred amount of a disintegrant in the tablet core, especially of CROSPOVIDONE, ranges from about 3.% to 5%, particularly from about 3.5% to 4.5%, by weight per unit dosage form.

A preferred amount of a glidant in the tablet core, especially of colloidal silicon dioxide, ranges from about 0.4 to 1.0%, particularly from about 0.6% to 0.9%, by weight per unit dosage form.

A preferred amount of a lubricant in the tablet core, especially of Mg stearate, ranges from about 1.5% to 3%, particularly from about 1.5% to 2.5% by weight per unit dosage form.

A preferred amount of a film coat ranges from about 0.4 mg/cm² to 0.7 mg/cm² per unit dosage form.

Preferred amounts of Aliskiren and additives are further shown in the illustrative examples.

The absolute amounts of each additive and the amounts relative to other additives is similarly dependent on the desired properties of the solid oral dosage form and may also be chosen by the skilled artisan by routine experimentation without undue burden. For example, the solid oral dosage form may be chosen to exhibit accelerated and/or delayed release of the active agent with or without quantitative control of the release of active agent.

In a specific embodiment of the invention, microcrystalline cellulose is used as a filler/binder; Crospovidone as an disintegrant, Povidone as a filler/binder, ethanol with 5% isopropanol as a granulation liquid, which may later be removed during processing, Silica, colloidal anhydrous / Colloidal Silicon Dioxide as a glidant and magnesium stearate as the lubricant, particularly in a concentration a shown in the illustrative examples.

In particular, the magnesium stearate in the external phase may be present in a concentration range of between about 1.5% to 3%, particularly from about 1.5% to 2.5% by weight per unit dosage form.

The cores of the minitablet according to the present invention and as described herein are coated with a coating, wherein said coating may serve to mask the bitter taste of the drug substance and therefore improve patient compliance and/or to control the release of the Aliskiren active compound *in vitro* and *in vivo.*

In one variant the minitablets are coated with a taste-masking material, e.g. a polyacrylate, preferably an Eudragit^{R} such as Eudragit^{R}-E or Eudragit^{R}-RD100 or -RS/RL (see Handbook of Pharmaceutical Excipients, loc. cit. hereafter, p. 362), especially Eudragit^{R}-E.

Suitable coating materials for the compositions of the invention include polyacrylates, especially polymethacrylates, preferably:
1. a) a copolymer formed from monomers selected from methacrylic acid, methacrylic acid esters, acrylic acid and acrylic acid esters;
2. b) a copolymer formed from monomers selected from butyl methacrylate, (2-dimethylaminoethyl)methacrylate and methyl methacrylate; or
3. c) a copolymer formed from monomers selected from ethyl acrylate, methyl methacrylate and trimethylammonioethyl methacrylate chloride;
more preferably those mentioned in 2.b), e.g. those available from Evonik under the trademark Eudragit^{R};

Especially preferred polyacrylic polymers are:
1. 1) the 1:1 copolymers formed from monomers selected from methacrylic acid and methacrylic acid lower alkyl esters, such as the 1:1 copolymers formed from methacrylic acid and methyl methacrylate available under the trademark Eudragit^{R} L, e.g. Eudragit^{R} L100, and the 1:1 copolymer of methacrylic acid and acrylic acid ethyl ester available under the trademark Eudragit^{R} L100-55;
2. 2) the 1:2:1 copolymer formed from butyl methacrylate, (2-dimethylaminoethyl)-methacrylate and methyl methacrylate available under the trademark Eudragit^{R} E; and
3. 3) the 1:2:0.2 copolymer formed from ethyl acrylate, methyl methacrylate and trimethylammonioethyl methacrylate chloride available under the trademark Eudragit^{R} RL; or the corresponding 1:2:0.1 copolymer available under the trademark Eudragit^{R} RS; or the 1:2:0.2 copolymer formed from ethyl acrylate, methyl methacrylate and trimethylammonioethyl methacrylate chloride which is in combination with carboxymethyl cellulose and available under the trademark Eudragit^{R} RD;
more preferably those mentioned in 2.2).

The polyacrylates above have preferably a mean molecular weight of about 50'000 to about 500'000, e.g. about 150'000.

It has been found that polyacrylates, especially Eudragit^{k} E, are particularly suitable for coating solid dosage forms comprising aliskiren in the form of the free base as well as in form of its salts, e.g. aliskiren hemifumarate, e.g. since a coating with Eudragit^{R} E does not easily dissolve at the neutral pH of the mouth, but only at pH values below 5, and thereby prevents the dissolution of the bitter tasting of aliskiren until transfer to the stomach.

Coating materials as hereinabove defined may be used in admixture with further excipients conventional in coating formulations, for example talcum, magnesium stearate or silicon dioxide, for example synthetic amorphous silicic acid of the Syloid^{R} type (Grace), for example Syloid^{R} 244 FP, or colloidal silicon dioxide, e.g. Aerosil^{R}, e.g. Aerosil^{R} 200, or wetting agents, for example sodium dodecyl sulfate or the aforementioned polyethyleneglycols or polysorbates.

In another aspect the solid dosage forms may comprise a further coating, e.g. a layer of anti-sticking material applied upon one of the above-mentioned coatings, e.g. comprising a colloidal silicon dioxide product, e.g. Aerosil^{R}, which may avoid adhesion of the solid dosage forms to each other or to the walls of the container material, e.g. a capsule.

Especially preferred compositions of the invention are coated aliskiren minitablets wherein the coating comprises a (taste-masking) polyacrylate coating, preferably Eudragit^{R} E or Eudragit RD100^{R}, especially Eudragit^{R} E.

The coating may further comprise further components such as a plasticizer, e.g. triacetine, triethylcitrate, diethylsebacate, polyethyleneglycol 3000, 4000 or 6000, acetyltriethylcitrate, acetyltributylcitrate, or diethylphthalate, and/or antisticking agents, e.g. magnesium stearate, colloidal silicon dioxide, an synthetic amorphous silicic acid such as Syloid 244 FP, talc, or glycerine monostearate. The coating may further comprise, especially in aqueous dispersions, one or more thickening agents to avoid sedimentation of suspended excipients, e.g. HPMC 3cps or HPMC 6 cps.

In a specific embodiment of the invention, magnesium stearate is used as an anti-tack agent, basic butylated methacrylate copolymer as a film forming agent, dibutyl sebacate as a plasticizer, sodium laurilsulfate / sodium lauryl sulphate as a solubilizing agent and purified water as a solvent (variant A).

In another variant the minitablets are coated with modified release coating components selected from cellulose derivatives; e.g. ethylcellulose, e.g. Aquacoat® ECD, available from FMC; Surelease available from Colorcon, acrylic copolymers, preferably acrylic and methacrylic copolymers containing quaternary ammonium groups, e.g. tri(C₁₋₄alkyl)-ammonium methylmethacrylate groups, e.g. trimethylammonium methylmethacrylate groups, e.g. acrylic/ methacrylicacid-ester with different ratio of quarternary ammonium groups 20:1 RL/ 40:1 RS, e.g. such polymers commercially available from Röhm Pharma under the Trademarks, Eudragit RL^{R}, Eudragit RS^{R} or Eudragit NE^{R} or copolymers; and/or mixtures thereof. A ratio of about 75:25, preferably 90:10, preferably 95:5 by weight Eudragit RS^{R}:Eudragit RL^{R} is particularly preferred.

The modified release coating components may be in aqueous dispersion, e.g. as 30% aqueous dispersion, or organic solution, e.g. 12.5% organic solution. For example the modified release coating components is a mixture of Eudragit RL^{R} and Eudragit RS^{R} in 30% aqueous dispersion or 12.5% organic solution.

The amount of modified release coating components may be from about 30 to about 100 weight %, more preferably from about 50 to about 100 weight %, based on the total weight of the coating.

The modified release coating, e.g. diffusion coating, preferably comprises 1 to 50 weight %, more preferably 2-20 weight %, even more preferably 4-10 weight %, of the total weight of the composition.

The skilled person would adjust the nature and amount of modified release coating polymer to adjust as necessary the dissolution profile of the Aliskiren component.

In another specific embodiment of the invention, a delayed release coating is provided, wherein dibutyl sebacate is used as a plasticizer, ethylcellulose aqueous dispersion as a film forming agent, hypromellose as another film forming agent, silica, colloidal anhydrous / colloidal silicon dioxide as a glidant and purified water as a solvent (variant B).

The modified release coating may further include one or more further components or excipients, e.g. pore formers, a plasticizer, an antisticking agent, a wetting agent, e.g. as disclosed hereinafter.

Suitable pore-formers may be pH independent pore-formers, such as HPMC, or pore-formers which are pH dependent, Suitable pH dependent pore-formers may be enteric pore-formers, e.g. enteric coating polymers.

As herein defined, an enteric pore-former is a pore-former which provides drug release in an environment with pH > 5, e.g. in intestinal fluid, and suppresses drug release in acidic environment, e.g. in the stomach. Example of enteric pore-formers according to the present invention are HPMC-phthalate (HPMC-P), e.g. HP50, HP55, e.g. from ShinEtsu; HPMC-acetate-succinate (HPMC-AS), e.g. Aqoat LF or Aqoat MF, e.g. from ShinEtsu; Methyl acrylic acid-ethyl acrylic acid copolymer, e.g. Methacrylic acid copolymer, e.g. Eudragit L, S, L100-55 and/or L30D from Röhm Pharma, Acryl-Eze from Colorcon, Kollicoat MAE 30 DP from BASF; Celluloseacetatephthalate, e.g. Aquacoat CPD from FMC Biopolymer, or Polymer from Eastman Kodak; and Polyvinylacetatephthalate, e.g. Sureteric, Colorcon, or any mixture thereof. Preferably HPMC-P and HPMC-AS may be combined with ethylcellulose or acrylic and methacrylic copolymers containing quaternary ammonium groups, e.g. tri(C₁₋₄alkyl)-ammonium methylmethacrylate groups, e.g. Eudragit RS in organic coating solutions, HPMC-AS dispersed in water can also be combined with aqueous ethylcellulose dispersion e.g. Aquacoat ECD, FMC.

Hydroxypropyl methylcellulose phthalates, typically have a molecular weight of from 20,000 to 100,000 Daltons e.g. 80,000 to 130,000 Daltons, e.g. a hydroxypropyl content of from 5 to 10%, a methoxy content of from 18 to 24% and a phthalyl content from 21 to 35%. Examples of suitable hydroxypropyl methylcellulose phthalates are the marketed products having a hydroxypropyl content of from 6-10%, a methoxy content of from 20-24%, a phthalyl content of from 21-27%, a molecular weight of about 84,000 Daltons known under the trade mark HP50 and available from Shin-Etsu Chemical Co. Ltd., Tokyo, Japan, and having a hydroxypropyl content, a methoxy content, and a phthalyl content of 5-9%, 18-22% and 27-35% respectively, and a molecular weight of 78,000 Daltons, known under the trademark HP55 and available from the same supplier.

Examples of suitable hydroxypropylmethylcellulose acetate succinate may be used as known under the trademark Aqoat LF or Aqoat MF and commercially available, e.g. from Shin-Etsu Chemical Co. Ltd., Tokyo, Japan.

The modified release coating of the composition of the invention may comprise 0 to 70 weight %, more preferably 5 to 50 weight % of pore-former, based on the total weight of the modified release coating.

The composition of the invention may further include a pore-former, e.g. which gives water-soluble pores, e.g. polyethyleneglycol, polyvinylpyrrolidone, polyethylene oxide, a cellulose derivative, e.g. hydroxyethyl cellulose, Hydroxypropylmethylcellulose (HPMC), Hydroxypropylcellulose, or other cellulose derivatives, e.g. which are soluble in acidic medium, e.g. as ammonium salt, acrylate or methacrylate esters, e.g., Eudragit E or Eudragit EPO; polyacrylic acid; which are swelling in water, e.g. Eudragit RS, RL, NE 30D, which are soluble in alkaline medium, i.e. enteric coating polymer, e.g. Eudragit L, S, L100-55 or any mixture thereof. HPMC may also act as a thickening agent due to the viscosity of the aqueous solution thereof. According to the invention the pore formers may be hydrophilic agents, e.g. water soluble plasticizers, e.g. PEG, triacetine, triethylcitrate, or hydrophilic silicium dioxide, e.g. Aerosil 200 or Syloid 244 FP.

Suitable plasticizers according to the invention include e.g., triacetine, triethy citrate, tributyl citrate, dibutylsebacate, diethyl sebacate, polyethyleneglycol 400, 3000, 4000 or 6000, acetyltriethylcitrate, acetyltributylcitrate and diethylphthalate, or mixtures thereof. Preferably the plasitcizer is triethylcitrate or dibutylsebacate. A plasticizer generally swells the coating polymer such that the polymer's glass transition temperature is lowered, its flexibility and toughness increased and its permeability altered. When the plasticizer is hydrophilic, such as polyethylene glycol, the water permeability of the coating is generally increased. When the plasticizer is hydrophobic, such as diethyl phthalate or dibutyl sebacate, the water permeability of the coating is generally decreased.

Preferably the plasticizer is present in an amount of 1 to 50% by weight, preferably 2 to 35%,more preferable 5-25% based on the total weight of the coating.

Examples of antisticking agents are silicon dioxide, e.g. colloidal silicon dioxide, an synthetic amorphous silicic acid such as Syloid 244 FP, talc, Aerosil 200 or glycerine monostearate. Preferably the antisticking agent is Areosil 200 and Syloid 244 FP. When the antisticking agent is hydrophilic, such as Aerosil 200 or Syloid 244 FP, the water permeability/swelling (and therefore also drug release) of the coating is generally increased. When the plasticizer is hydrophobic, such as talcum or glycerolmonostearate, the water permeability of the coating is generally decreased. Antisticking agents are optionally included in the coating formulation to avoid sticking of the drug cores and guarantee a high separation of them.

Preferably the antisticking agent is present in an amount of 1 to 50% by weight, more preferably 5 to 25% by weight, based on the total weight of the coating.

Suitable wetting agents include e.g. sodium laurylsulphate, cetomacrogol, a wax, glycerol monostearate, a sorbitan ester and a poloxamer. Wetting agents are optionally included in the coating formulation due to their property to reduce interfacial tensions and improve the contact of spray solutions or suspensions with treated surfaces.

Preferably the wetting agent is present in an amount of 0.1 to 20% by weight, more preferably 1 to 5% by weight, based on the dry weight of the coating.

In still another specific embodiment of the invention, a slow release coating is provided, wherein silica, colloidal anhydrous / colloidal silicon dioxide is used as a glidant, triethyl citrate as a plasticizer, ammonio methacrylate copolymer type A as a film forming agent, ammonio methacrylate copolymer type B as another film forming agent and purified water as a solvent (variant C).

For all three variants A, B and C, it could surprisingly be shown that the bioavailability of the Aliskiren compound could be substantially maintained at a level comparable to that of known solid dosage forms such as the marketed Aliskiren product or a capsule formulation, the bioavailability of which was shown in previous clinical studies to be significantly higher than that of liquid Aliskiren formulations. Thus, it was a surprising finding that with the formulation according to the present invention a faster dissolution rate than for solid dosage forms could be achieved, similar to that seen with liquid formulations, but without compromising on the bioavailability which is comparable to that of known solid dosage forms and improved compared to liquid formulations.

The solid unit dosage form according to the invention and as described herein, particularly the solid unit dosage form of Aliskiren for oral administration in form of coated minitablets, has a bioavailability which is significantly higher than that of liquid Aliskiren formulations. In particular, bioavailability of the coated minitablets is enhanced as compared to liquid Aliskiren formulations by a factor of at least 1.1, particularly by a factor of at least 1.3, but especially by a factor of at least 1.5.

This is especially surprising for the solid unit dosage form according to variant A, which shows a very quick release of the active ingredient after administration comparable to that of liquid presentations.

For the preparation of the core tablet comprising a therapeutically effective amount of Aliskiren, or a pharmaceutically acceptable salt thereof, wet granulation may be used.

Wet granulation of Aliskiren with excipients using water and/or an aqueous binder solution leads to a change in polymorphism of the drug substance which changes partly to the amorphous state and causes an inferior chemical stability of the drug product (DP). However, wet granulation of Aliskiren using a mixture of organic solvents or an organic binder solution has been found to be the best way of manufacturing suitable Aliskiren solid oral dosage forms, especially tablets, showing following advantages:
- Said wet granulation reduces the bulk volume of Aliskiren during granulation;
- The influences of a changing drug substance quality are minimized;
- A high drug loading above 46% by weight per unit dosage form may easily be achieved;
- The formulation of tablets with sufficient hardness, resistance to friability, disintegration time, dissolution rate etc. is possible;
- The sticking tendency and poor flow of the drug substance is reduced to a minimum;
- A robust manufacturing process of the DP is achieved;
- Scale-up of formulation and process resulting in a reproducible DP performance is achieved; and
- Sufficient stability to achieve a reasonable shelf life is achieved.

The excipients may be distributed partly in the inner (granular) phase and partly in the outer phase, which is the case in the described invention. Microcrystalline cellulose (filler) and CROSPOVIDONE (disintegrant) are partly in the inner and partly in the outer phase, PVP K 30 (binder) is only part of the inner phase, being the binder during granulation, whereas colloidal silicon dioxide (glidant) and Mg stearate (lubricant) are only part of the outer phase.

The inner phase excipients, e.g., filler, binder and disintegrant, and the drug substance are mixed and granulated with an ethanolic solution of the binder and additional ethanol. The granulate is dried and sieved. The outer phase containing, e.g., disintegrant, filler, glidant and lubricant, is screened with the dried granulate and mixed. The mixture is compressed into tablets. The cores are coated with a film-coat.

The granulate phase is defined as the inner phase, the excipients added to the granulate are defined as the outer phase of the tabletting mixture.

A process for the manufacture of a solid oral dosage form of the present invention comprises:
1) mixing the active ingredient and additives and granulating said components with a granulation liquid;
2) drying a resulting granulate with intervening screening steps;
3) mixing the dried granulate with outer phase excipients;
4) compressing a resulting mixture to form a solid oral dosage as a core tablet; and
5) coating a resulting core tablet to give a film-coated tablet.

Preferably, the additives in step (1) are selected from a filler, a disintegrant and a binder; and the outer phase excipients in step (3) are selected from a filler, a disintegrant, a lubricant and a glidant.

The granulation liquid can be ethanol, a mixture of ethanol and water, a mixture of ethanol, water and isopropanol, or a solution of PVP in the before mentioned mixtures. A preferred mixture of ethanol and water ranges from about 50/50 to about 99/1 (% w/w), most preferably it is about 94/6 (% w/w). A preferred mixture of ethanol, water and isopropanol ranges from about 45/45/5 to about 98/1/1 (% w/w/w), most preferably from about 88.5/5.5/6.0 to about 91.5/4.5/4.0 (% w/w/w). A preferred concentration of PVP in the above named mixtures ranges from about 5 to about 30% by weight, preferably from about 15 to about 25%, more preferably from about 16 to about 22%.

Attention is drawn to the numerous known methods of granulating, drying and mixing employed in the art, e.g., spray granulation in a fluidized bed, wet granulation in a high-shear mixer, melt granulation, drying in a fluidized-bed dryer, mixing in a free-fall or tumble blender, compressing into tablets on a single-punch or rotary tablet press.

The manufacturing of the granulate can be performed on standard equipment suitable for organic granulation processes. The manufacturing of the final blend and the compression of tablets can also be performed on standard equipment.

For example, step (1) may be carried out by a high-shear granulator, e.g., Collette Gral; step (2) may be conducted in a fluid-bed dryer; step (3) may be carried out by a free-fall mixer (e.g. container blender, tumble blender); and step (4) may be carried out using a dry compression method, e.g., a rotary tablet press.

Due to the high hygroscopicity and water sensitivity of Aliskiren with respect to changes in polymorphism, the use of water has preferably to be avoided in order to prevent the drug substance from changes in polymorphism for the above stated reasons (amorphous state, inferior chemical stability). A solution for said problem is to apply an organic film-coating process.

Surprisingly it was found that an aqueous film coating process using a standard film-coat composition can be applied to Aliskiren core tablets without changes in polymorphism.

The film-coat may also comprise HPMC as the polymer, iron oxide pigments, titanium dioxide as coloring agent, PEG as softener and talc as anti-tacking agent. The use of coloring agents or dyes may serve to enhance the appearance as well as to identify the compositions. Other dyes suitable for use typically include carotinoids, chlorophyll and lakes.

The film coating conditions have to assure that the tablet cores do not take up considerable amounts of moisture and that the drug substance within the tablets does not closely get into contact with water droplets. This is achieved by process parameter settings that reduce the amount of humidity which gets onto the tablet cores.

The manufacture of minitablets can therefore be based on existing granulate used in the manufacture of the standard dosage form, however adaptations to formulation and manufacturing process are necessary.

Minitablets are compressed on a standard rotary tablet press with special multi-tip tooling. Multi-tip tooling can consist of up to 19 tips per punch. Such minitablet punches have a larger contact area in the die compared with a standard tablet punch. Therefore the lubrication of the formulation and / or tooling (e. g. spray lubrication) options plays an important role in the manufacturing process.

During the development trials lower punch stiffness was observed despite low ejection forces. To overcome this problem, a different punch tip design and a special filling cam on the rotary tablet press was used. Adaptations to the formulation, e. g. increase of lubricant concentration may also be made.

In a further step the dissolution rate behavior of the minitablets is aligned to that of the mono tablets by modifying the release through application of a film coating.

In a specific embodiment of the present invention, three variants are provided produced from the same mixture of granulate used in the production of the SPP100 marketed formulation, wherein the outer phase is modified through application of different coatings. The variants are described as:
Variant A: pH-sensitive Eudragit EPO coat, fastest DR
Variant B: Ethylcellulose + HPMC coat, medium DR
Variant C: Eudragit RL/RS coat, slowest DR

In order to overcome the adverse concurrence of high surface area of minitablets coupled with hygrospcopicity of Aliskiren and the problems associated therewith, the drying conditions in the pan are to be modified in order to reduce the high water content in the film-coated minitablets. This may be achieved by using a coating instrument with improved drying conditions such as, for example, a fluidized bed coater.

In an additional step, the external phase may also be modified by removing the disintegrant, which is hygroscopic in nature. This is possible, because the removal of the disintegrant can be compensated through the high surface area provided by the minitablets resulting in faster disintegration even without disintegrant. Further, the amount of lubricant may be increased as compared with the marketed dosage form because the multi-tip tooling used in the manufacture of the minitablets has large contact area.

The solid oral dosage forms of the present invention can be used in pediatrics for lowering the blood pressure, either systolic or diastolic or both in children. The conditions for which the instant invention is useful include, without limitation, hypertension (whether of the malignant, essential, reno-vascular, diabetic, isolated systolic, or other secondary type), congestive heart failure, angina (whether stable or unstable), myocardial infarction, atherosclerosis, diabetic nephropathy, diabetic cardiac myopathy, renal insufficiency, peripheral vascular disease, left ventricular hypertrophy, cognitive dysfunction (such as Alzheimer's) and stroke, headache and chronic heart failure.

The present invention likewise relates to a pharmaceutical composition for the pediatric treatment of hypertension (whether of the malignant, essential, reno-vascular, diabetic, isolated systolic, or other secondary type), congestive heart failure, angina (whether stable or unstable), myocardial infarction, atherosclerosis, diabetic nephropathy, diabetic cardiac myopathy, renal insufficiency, peripheral vascular disease, left ventricular hypertrophy, cognitive dysfunction, stroke, headache and chronic heart failure, comprising a solid oral dosage form according to the present invention.

Ultimately, the exact dose of the active agent and the particular formulation to be administered depend on a number of factors, e.g., the condition to be treated, the desired duration of the treatment and the rate of release of the active agent. For example, the amount of the active agent required and the release rate thereof may be determined on the basis of known *in vitro* or *in vivo* techniques, determining how long a particular active agent concentration in the blood plasma remains at an acceptable level for a therapeutic effect.

The above description fully discloses the invention including preferred embodiments thereof. Modifications and improvements of the embodiments specifically disclosed herein are within the scope of the following claims. Without further elaboration, it is believed that one skilled in the art can, using the preceding description, utilize the present invention to its fullest extent. Therefore, the Examples herein are to be construed as merely illustrative and not a limitation of the scope of the present invention in any way.

### Examples

### Example 1: Preparation of Minitablet Variants

Three pediatric minitablet variants (300 mg SPP100 minitablets (3.125 mg/tablet)) were produced from the same mixture of granulate that is also used in the SPP100 marketed formulation. Modifications were made to the outer phase of the core tablet and a coating was applied resulting in the following variants:
1. Variant A: pH-sensitive Eudragit EPO coat, fastest DR
2. Variant B: Ethylcellulose + HPMC coat, medium DR
3. Variant C: Eudragit RL/RS coat, slowest DR

The composition of the above variants is described in tables 1-1, 1-2 and 1-3.

### Example 2: Relative BA Study

A relative BA study was designed to evaluate formulation performance of the pediatric minitablet variant A (300 mg SPP100 minitablets (3.125 mg/tablet)). The composition of the investigational drug is given in Tables 1-1. SPP100 300 mg Final Market Image (FMI) tablets as marketed were used as a comparative standard.

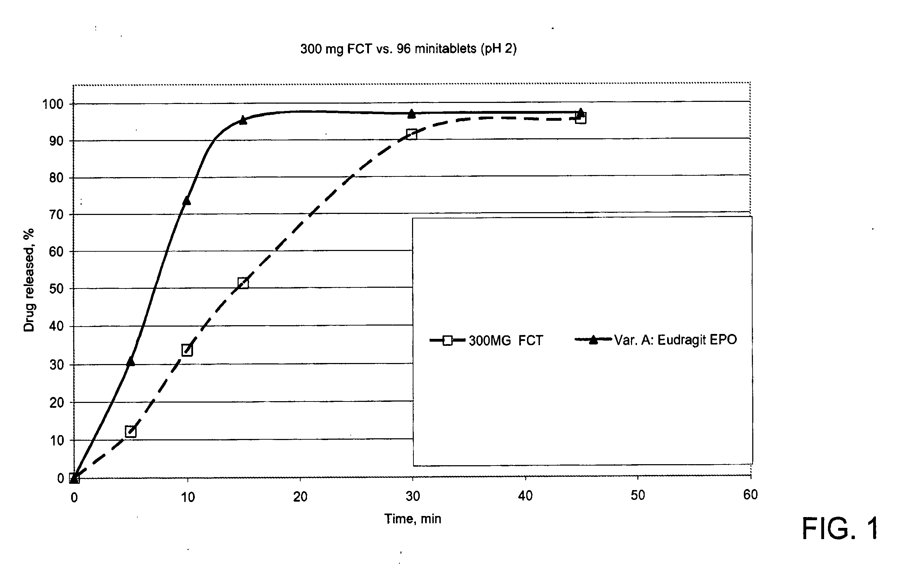

Pharmacokinetic parameters as well as taste were measured in adult volunteers. The results of these studies for Variant A are provided in Tables 2 to 3 and in Figure 1.

### Reference Example 1: Aliskiren Capsule

composition [mg] of the capsule fill (capsule #0):

| | |
|---|---|
| Aliskiren hemifumarate | 41.44 |
| Emcocel 50M | 171.1 |
| Polyplasdone XL | 26.56 |
| Aerosil 200 | 2.4 |
| Magnesiumstearat | 3.6 |
| Sum | 245.1 |

The capsule is filled with a dry blend of the drug substance and the excipients. A dose of 75 mg aliskiren base is administered as two capsules.

### Reference Example 2: Liquid Aliskiren formulation

The equivalent of 75mg aliskiren base (= 82.8 mg hemifumarate) drug substance is filled into a 125ml bottle without any additional excipients and is then administered with 200ml of tap water whereby the drug substance is dissolved in the bottle by one part of water and the remainder of the water is used to rinse the bottle.

### Example 3: Pharmacokinetics in Pediatric Patients

An open label, multiple dose pharmacokinetic and pharmacodynamic study was performed in 24 pediatric patients, ages 6-17 years. Single daily doses of aliskiren at either 2 mg/kg or 6 mg/kg dose level were administered for eight consecutive days. Blood samples were collected on days one and eight and pharmacokinetics were assessed using a non-compartmental analysis approach.

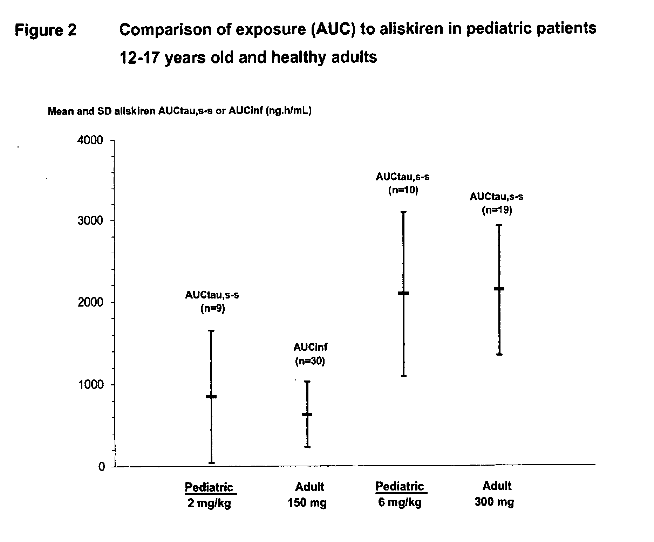

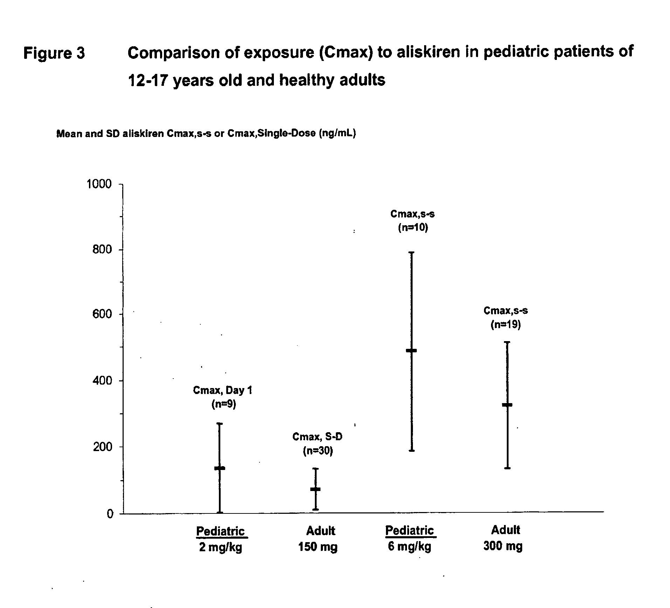

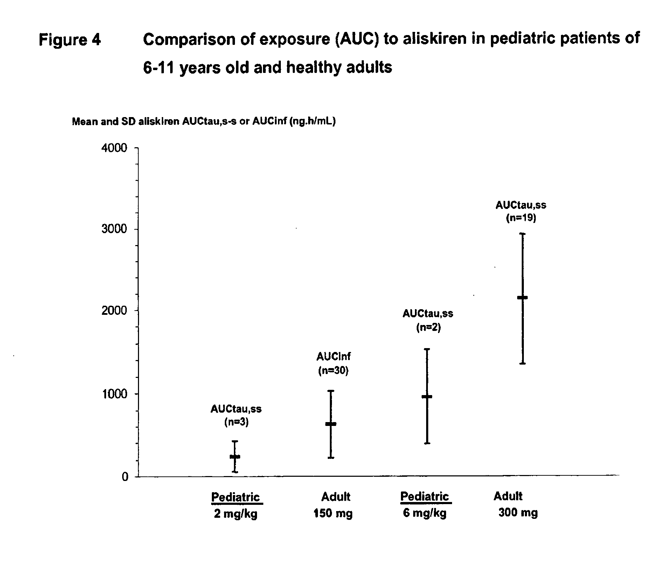

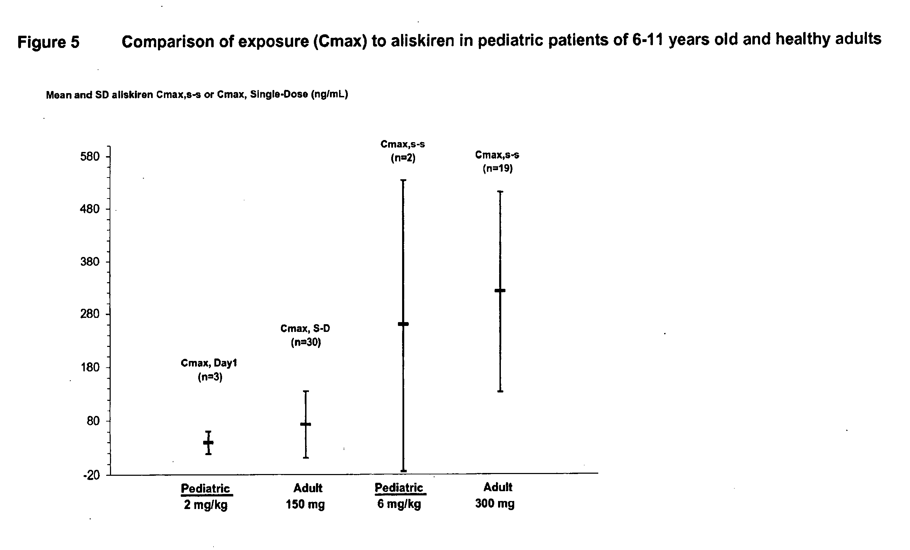

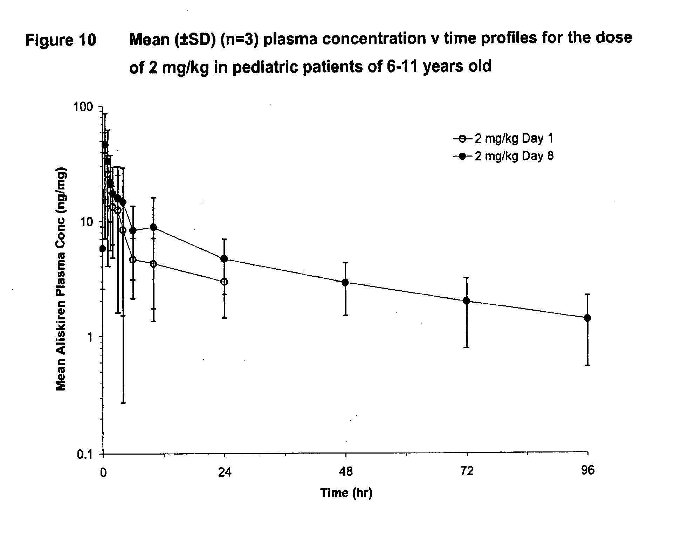

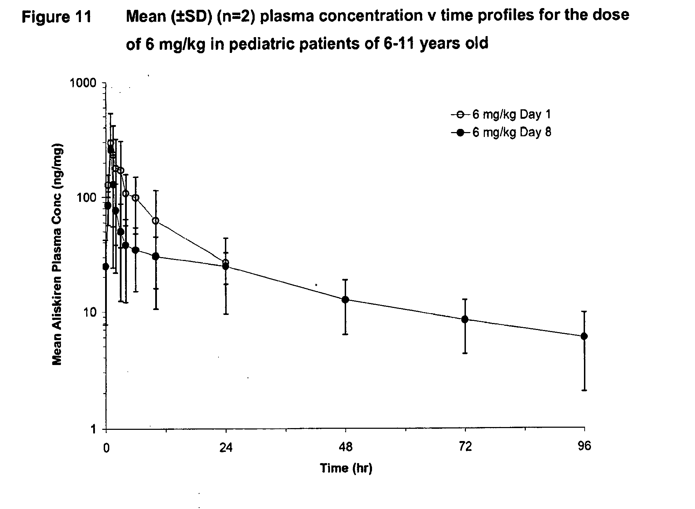

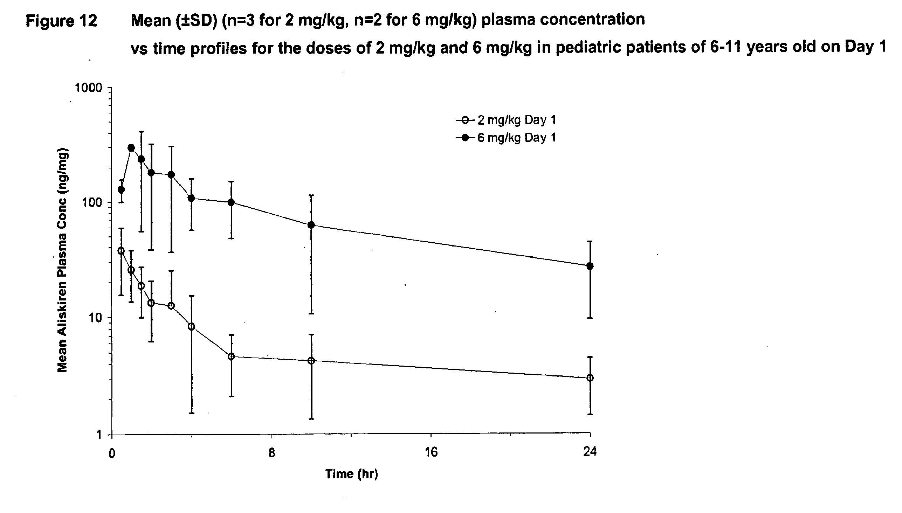

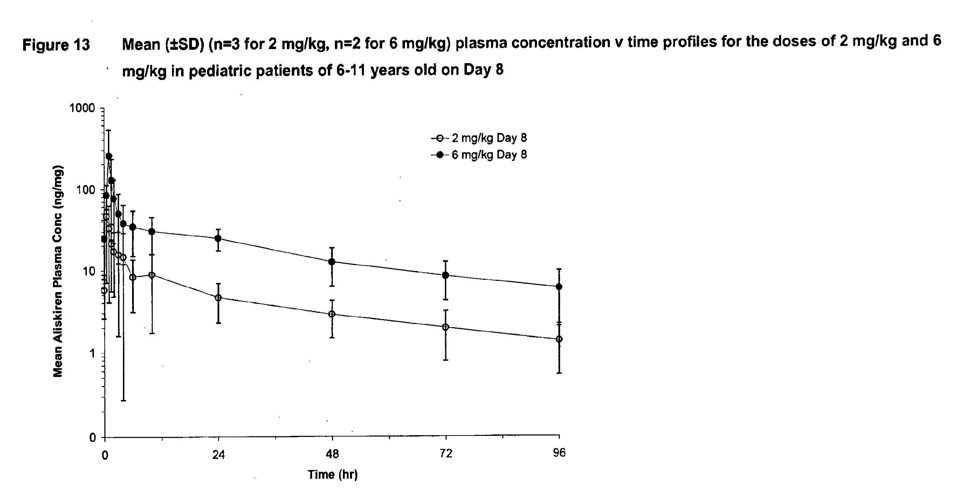

The exposure to aliskiren in pediatric patients was observed to be similar to the exposure found in healthy adults at comparable doses. The results are shown in Table 5 and Figures 2-13. The mean drug exposure (AUC and Cₘₐₓ) at both 2 mg/kg and 6 mg/kg, was similar to the exposures observed in healthy adults (Figs. 2-5) but with greater variability, likely due to a smaller sample size (12-17 years: n = 19 total, n = 9 at 2 mg/kg and n = 10 at 6 mg/kg; 6-11 years: n = 5 total, n = 3 at 2 mg/kg and n = 2 at 6 mg/kg). The median Tₘₐₓ, mean drug accumulation index and terminal elimination half lives were consistent with those observed in adults.

Overall drug accumulation in patients 12-17 years of age was low to moderate, when mean exposure curves on Day 8 were compared with those on Day 1. A reasonable exposure increase was observed in response to dosage increments, comparing the mean exposure curves of 6 mg/kg dose and 2 mg/kg. At steady state (Day 8), in patients 12-17, a three-fold increase in dose resulted in a 2.5 fold increase in AUC and a 1.7 fold increase in Cₘₐₓ. These findings are consistent with aliskiren pharmacokinetics in adult subjects. The results in patients 6-11 years old were similar to those observed in adults and the older pediatric cohort.

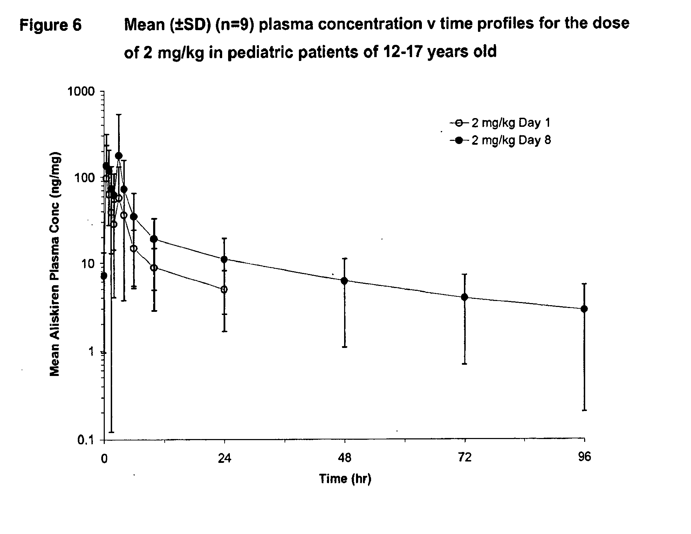

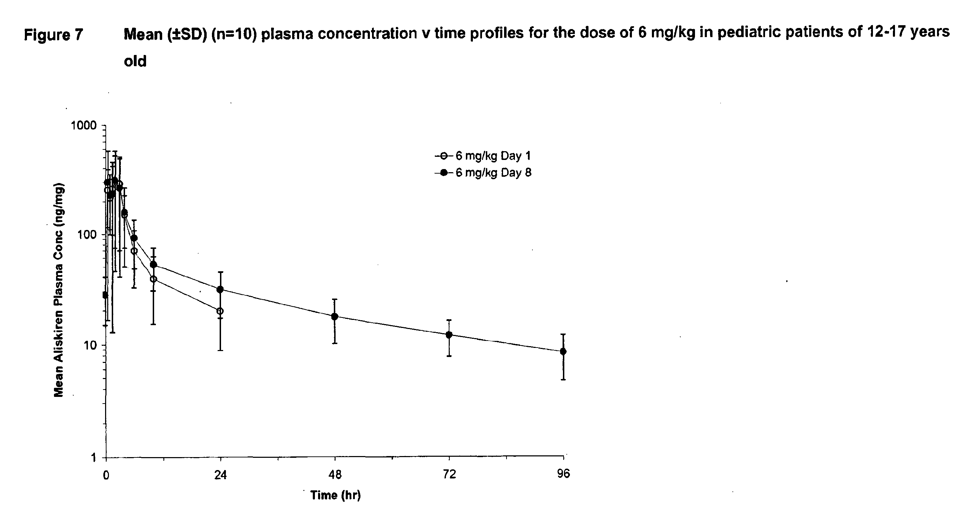

The mean concentration-time profiles on days one and eight in pediatric patients 12-17 years old at 2 mg/kg is shown in Figure 6 and at 6 mg/kg is shown in Figure 7.

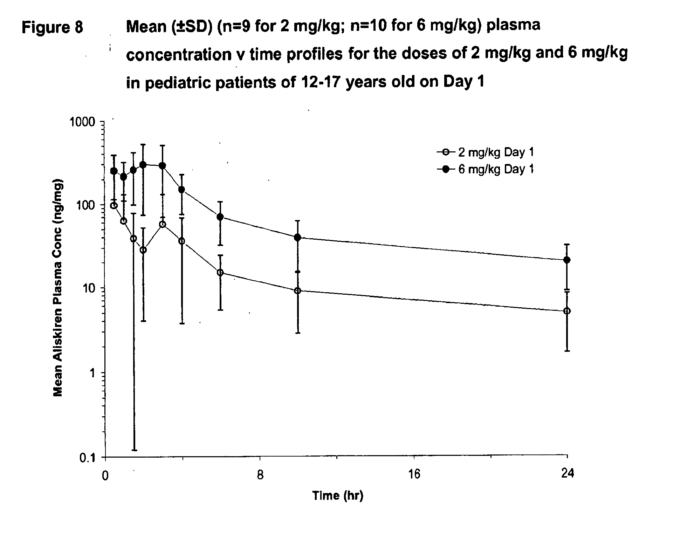

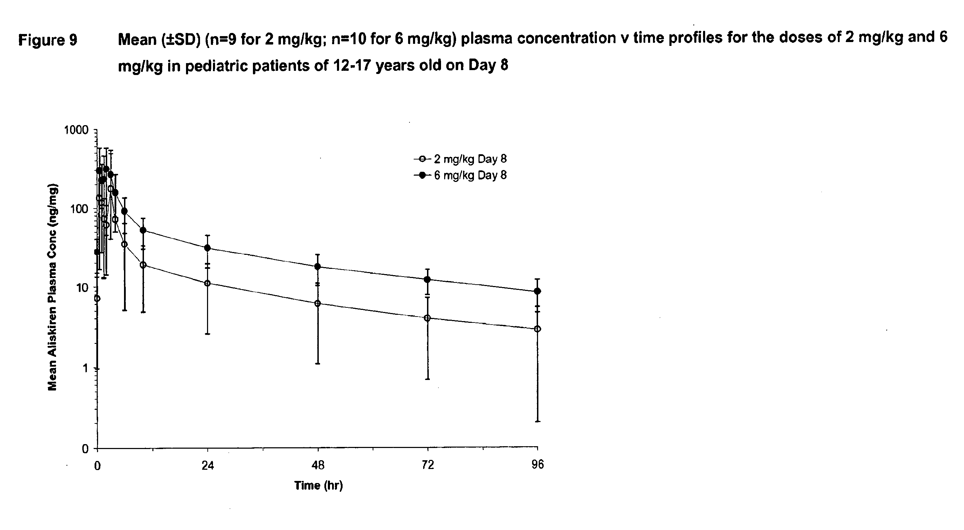

The mean concentration-time profile on day one in pediatric patients 12-17 years old at doses of 2 mg/kg (n=9) and 6 mg/kg (n=10) is shown in Figure 8. The mean concentration-time profile on day eight in pediatric patients 12-17 years old at 2 mg/kg (n=9) and at 6 mg/kg (n=10) is shown in Figure 9.

Figure 10 shows the mean concentration-time profile on days one and eight in pediatric patients 6-11 years old at a dose of 2 mg/kg (n=3). Figure 11 shows the mean concentration-time profile on days one and eight in pediatric patients 6-11 years old at a dose of 6 mg/kg (n=3).

Figure 12 shows the mean concentration-time profile on day one in pediatric patients 6-11 years old at doses of 2 mg/kg (n=3) and 6 mg/kg (n=2). Figure 13 shows the mean concentration-time profile on day eight in pediatric patients 6-11 years old at doses of 2 mg/kg (n=3) and 6 mg/kg (n=2).

### TABLES

**Table 1-1 Composition of SPP100 3.125 mg film-coated 'mini' tablet; coated with a film-coating based on basic butylated methacrylate copolymer (Variant A)**

| **Ingredient** | **Amount per 3.125 mg film-coated tablet (mg)** | **Function** |
|---|---|---|
| **Tablet core** | | |
| SPP100 hemifumarate | 3.4531 * | Active ingredient |
| Cellulose microcrystalline / Microcrystalline Cellulose | 2.613 | Filler/Binder |
| Crospovidone | 0.296 | Disintegrant |
| Povidone | 0.250 | Filler/Binder |
| Ethanol with 5% Isopropanol | --- ** | Granulation liquid |
| Silica, colloidal anhydrous / Colloidal Silicon Dioxide | 0.060 | Glidant |
| Magnesium stearate | 0.127 | Lubricant |
| **Core tablet weight** | 6.799 | |
| **Coating** | Approx. amounts (mg) | |
| Magnesium stearate | 0.09 | Anti-tack |
| Basic butylated methacrylate copolymer | 0.35 | Film forming agent |
| Dibutyl sebacate | 0.05 | Plasticizer |
| Sodium laurilsulfate / Sodium lauryl sulphate | 0.02 | Solubilizing agent |
| Water, purified** | ---** | Solvent |
| **Total film-coated tablet weight** | 7.31 | |

| | | |
|---|---|---|
| *Corresponds to e.g. 3.125 mg SPP100 base **Removed during processing | | |

**Table 1-2 Composition of SPP100 3.125 mg film-coated 'mini' tablet; coated with a film-coating based on ethylcellulose and hypromellose (Variant B)**

| **Ingredient** | **Amount per 3.125 mg film-coated tablet (mg)** | **Function** |
|---|---|---|
| **Tablet core** | | |
| SPP100 hemifumarate | 3.4531 * | Active ingredient |
| Cellulose microcrystalline / Microcrystalline Cellulose | 2.613 | Filler/Binder |
| Crospovidone | 0.296 | Disintegrant |
| Povidone | 0.250 | Filler/Binder |
| Ethanol with 5% Isopropanol | --*** | Granulation liquid |
| Silica, colloidal anhydrous / Colloidal Silicon Dioxide | 0.060 | Glidant |
| Magnesium stearate | 0.161 | Lubricant |
| **Core tablet weight** | 6.833 | |
| **Coating** | Approx. amounts (mg) | |
| Dibutyl sebacate | 0.0976 | Plasticizer |
| Ethylcellulose Aqueous Dispersion | 0.208 | Film forming agent |
| Hypromellose | 0.254 | Film forming agent |
| Silica, colloidal anhydrous / Colloidal Silicon Dioxide | 0.040 | Glidant |
| Water, purified** | --- ** | Solvent |
| **Total film-coated tablet weight** | 7.433 | |
| *Corresponds to 3.125 mg SPP100 base | | |
| **Removed during processing | | |

**Table 1-3 Composition of SPP100 3.125 mg film-coated 'mini' tablet; coated with a film-coating based on ammonio methacrylate copolymer type A and B (Variant C)**

| **Ingredient** | **Amount per 3.125 mg film-coated tablet (mg)** | **Function** |
|---|---|---|
| **Tablet core** | | |
| SPP100 hemifumarate | 3.4531 * | Active ingredient |
| Cellulose microcrystalline / Microcrystalline Cellulose | 2.613 | Filler/Binder |
| Crospovidone | 0.296 | Disintegrant |
| Povidone | 0.250 | Filler/Binder |
| Ethanol with 5% Isopropanol | --- ** | Granulation liquid |
| Silica, colloidal anhydrous / Colloidal Silicon Dioxide | 0.0600 | Glidant |
| Magnesium stearate | 0.161 | Lubricant |
| **Core tablet weight** | 6.833 | |
| **Coating** | Approx. amounts (mg) | |
| Silica, colloidal anhydrous / Colloidal Silicon Dioxide | 0.076 | Glidant |
| Triethyl citrate | 0.060 | Plasticizer |
| Ammonio methacrylate copolymer type A | 0.142 | Film forming agent |
| Ammonio methacrylate copolymer type B | 0.142 | Film forming agent |
| Water, purified** | ---** | Solvent |
| **Total film-coated tablet weight** | 7.2531 | |
| *Corresponds to 3.125 mg SPP100 base | | |
| **Removed during processing | | |

**Table 2 Geometric mean (CV%) values of SPP Cmax and AUC of Variant A**

| | **SPP reference (N=29)** | | **Variant A (N=25)** | |
|---|---|---|---|---|
| | **Geo_mean** | **CV%** | **Geo_mean** | **CV%** |
| **Cmax (ng/mL)** | 186 | 55 | 289 | 44 |
| **AUC0-t (ng.h/mL)** | 1149 | 40 | 1412 | 45 |
| **AUC0-inf (ng.h/mL)** | 1262 | 40 | 1539 | 44 |

**Table 3 Geometric mean ratio and 90% CI of SPP Variant A**

| | **Var A / Ref** | |
|---|---|---|
| | **Pt est** | **90% CI** |
| **Cmax (ng/mL)** | 1.499 | 1.181 - 1.901 |
| **AUC0-t (ng.h/mL)** | 1.164 | 1.004 -1.350 |
| **AUC0-inf (ng.h/mL)** | 1.168 | 1.010 - 1.350 |

**Table 4 Geometric mean values of SPP Cmax and AUC of 75MG SPP100 solution**

| | **2x37.5MG SPP capsule (reference)** | **75MG SPP solution** |
|---|---|---|
| | **Geo_mean** | **Geo_mean** |
| **Cmax (ng/mL)** | 28 | 15 |
| **AUC0-inf (ng.h/mL)** | 225 | 158 |

The bioavailability of the formulation in accordance with the present invention had comparable bioavailability to the marketed Aliskiren tablet (reference) as well as the capsule formulation and much improved bioavailability compared to the liquid formulation.

**Table 5 Pharmacokinetics in Pediatric Patients**

| **Age group (yrs old)** | | **Dose** | **Day** | **Cmax (ng/mL)** | **Tmax* (hr)** | **AUCtau (ng.hr/mL)** | **Al** | **T1/2 (hr)** |
|---|---|---|---|---|---|---|---|---|
| **12-17** | **Mean** | **2 mg/kg** | **1** | **136** | **1.0** | **391** | -- | -- |
| | **SD** | | | 133 | 0.5, 3.0 | 264 | - | - |
| | **CV%** | | | 97 | | 68 | -- | - |
| | | | | | | | | |
| | **Mean** | | **8** | **279** | **1.0** | **846** | **2.1** | **40** |
| | **SD** | | | 358 | 0.5, 3.0 | 804 | 1.3 | 5.0 |
| | **CV%** | | | 128 | | 95 | 62 | 13 |
| | | | | | | | | |
| | **Mean** | **6 mg/kg** | **1** | **424** | **1.8** | **1801** | -- | -- |
| | **SD** | | | 189 | 0.5, 4.0 | 811 | -- | -- |
| | **CV%** | | | 44 | | 45 | -- | -- |
| | | | | | | | | |
| | **Mean** | | **8** | **486** | **2.0** | **2089** | **1.3** | **42** |
| | **SD** | | | 301 | 0.5, 3.0 | 1003 | 0.6 | 6.5 |
| | **CV%** | | | 62 | | 48 | 50 | 16 |
| | | | | | | | | |
| **6-11** | **Mean** | 2 **mg/kg** | **1** | **39** | **0.5** | **149** | -- | -- |
| | **SD** | | | 21 | 0.5, 3.0 | 74 | -- | -- |
| | **CV%** | | | **53** | | 49 | -- | -- |
| | | | | | | | | |
| | **Mean** | | **8** | **48** | **0.5** | **240** | **1.5** | **40** |
| | **SD** | | | 39 | 0.5, 1.0 | 183 | 0.5 | 6.6 |
| | **CV%** | | | 81 | | 76 | 31 | 16 |
| | | | | | | | | |
| | **Mean** | **6 mg/kg** | **1** | **323** | **1.3** | **1841** | -- | -- |
| | **SD** | | | 55 | 1.0, 1.5 | 1168 | -- | -- |
| | **CV%** | | | 17 | | 63 | -- | -- |
| | | | | | | | | |
| | **Mean** | | **8** | **259** | **0.8** | **956** | **0.5** | **36** |
| | **SD** | | | 274 | 0.5, 1.0 | 568 | 0.0 | 0.7 |
| | **CV%** | | | 106 | | 59 | 0 | 2 |

| | | | | | | | | |
|---|---|---|---|---|---|---|---|---|
| * Median (min, max) | | | | | | | | |

## Claims

1. A solid unit dosage form for oral administration in form of a minitablet having a core and an outer coating, wherein
- the core comprises a therapeutically effective amount of aliskiren, or a pharmaceutically acceptable salt thereof, and
- the outer coating is in form of a film-coat which comprises a taste masking material selected from polyacrylates, and/or a modified release coating component selected from cellulose derivatives and acrylic copolymers, and mixtures thereof,
wherein said minitablet has a size of between 1 mm and 4 mm, and wherein said minitablet contains between about 2 mg and about 4 mg aliskiren.

2. The solid unit dosage form according to claim 1, wherein said minitablet has a size of between 1.5 mm and 3 mm.

3. The solid unit dosage form according to claim 1, wherein said minitablet has a size of between 1.5 mm and 2.5 mm.

4. The solid unit dosage form according to claim 1, wherein said minitablet has a size of 2 mm.

5. The solid unit dosage form according to claim 4, wherein said minitablet contains 3.125 mg aliskiren.

6. The solid unit dosage form according to any of the preceding claims, wherein the polyacrylate is selected from
a) a copolymer formed from monomers selected from methacrylic acid, methacrylic acid esters, acrylic acid and acrylic acid esters;
b) a copolymer formed from monomers selected from butyl methacrylate, (2-dimethylaminoethyl)methacrylate and methyl methacrylate; and
c) a copolymer formed from monomers selected from ethyl acrylate, methyl methacrylate and trimethylammonioethyl methacrylate chloride.

7. The solid unit dosage form according to claim 6, wherein the polyacrylate is a copolymer formed from monomers selected from butyl methacrylate, (2-dimethylaminoethyl)methacrylate and methyl methacrylate.

8. The solid unit dosage form according to claim 6 or 7, wherein the film-coat comprises magnesium stearate, basic butylated methacrylate copolymer, dibutyl sebacate, and sodium lauryl sulphate.

9. The solid unit dosage form according to any of the preceding claims, wherein said film-coat has a pH-dependent release profile.

10. The solid unit dosage form according to claim 9, wherein said film-coat leads to an *in vitro* dissolution of aliskiren of about 30% or less after 5 minutes, of about 80% or less after 10 minutes, and of about 95% or less after 15 minutes, at a pH of between about 2 and 4.5, where the test for measuring the dissolution of the individual active ingredient is performed following pharmacopeia USP <711> at pH 2.0 using a basket method at 100 rotations per minute and the dissolution medium is a phosphate buffer.

11. The solid unit dosage form according to claim 10, wherein said film-coat comprises a basic butylated methacrylate copolymer.

12. The solid unit dosage form according to any of claims 1 to 5 wherein said film-coat comprises an ethylcellulose aqueous dispersion and, optionally, hypromellose.

13. The solid unit dosage form according to claim 12, wherein the film-coat comprises dibutyl sebacate, ethylcellulose aqueous dispersion, hypromellose, and colloidal silicon dioxide.

14. The solid unit dosage form according to any of claims 1 to 5, wherein said film-coat comprises an ammonium methacrylate copolymer, particularly an ammonium methacrylate copolymer type A and/or an ammonium methacrylate copolymer Type B.

15. The solid unit dosage form according to claim 14, wherein the film-coat comprises colloidal silicon dioxide, triethyl citrate, ammonium methacrylate copolymer type A and/or an ammonium methacrylate copolymer Type B.

16. A solid unit dosage form according to any of the preceding claims for use in the treatment of a disease or condition in a pediatric population.

17. A solid oral dosage form according to any one of the preceding claims for use in the treatment of hypertension, congestive heart failure, angina, myocardial infarction, atherosclerosis, diabetic nephropathy, diabetic cardiac myopathy, renal insufficiency, peripheral vascular disease, left ventricular hypertrophy, cognitive dysfunction, stroke, headache and chronic heart failure.

## Patentansprüche

1. Feste Einheitsdosierungsform zur oralen Verabreichung in Form einer Minitablette mit einem Kern und einer äußeren Beschichtung, wobei
- der Kern eine therapeutisch wirksame Menge an Aliskiren oder einem pharmazeutisch verträglichen Salz davon umfasst, und
- die äußere Beschichtung in Form einer Filmbeschichtung ausgeführt ist, die ein geschmacksmaskierendes Material, ausgewählt aus Polyacrylaten ,und/oder eine Beschichtungskomponente für modifizierte Freisetzung, ausgewählt aus Cellulosederivaten und Acrylcopolymeren und Mischungen davon, umfasst,
wobei die Minitablette eine Größe von zwischen 1 mm und 4 mm aufweist und wobei die Minitablette zwischen ungefähr 2 mg und ungefähr 4 mg Aliskiren enthält.

2. Feste Einheitsdosierungsform nach Anspruch 1, wobei die Minitablette eine Größe von zwischen 1,5 mm und 3 mm aufweist.

3. Feste Einheitsdosierungsform nach Anspruch 1, wobei die Minitablette eine Größe von zwischen 1,5 mm und 2,5 mm aufweist.

4. Feste Einheitsdosierungsform nach Anspruch 1, wobei die Minitablette eine Größe von 2 mm aufweist.

5. Feste Einheitsdosierungsform nach Anspruch 4, wobei die Minitablette 3,125 mg Aliskiren enthält.

6. Feste Einheitsdosierungsform nach einem der vorangehenden Ansprüche, wobei das Polyacrylat ausgewählt ist aus
a) einem Copolymer gebildet aus Monomeren, ausgewählt aus Methacrylsäure, Methacrylsäureestern, Acrylsäure und Acrylsäureestern;
b) einem Copolymer gebildet aus Monomeren, ausgewählt aus Butylmethacrylat, (2-Dimethylaminoethyl)-Methacrylat und Methylmethacrylat; und
c) einem Copolymer gebildet aus Monomeren, ausgewählt aus Ethylacrylat, Methylmethacrylat und Trimethylammonioethyl-Methacrylatchlorid.

7. Feste Einheitsdosierungsform nach Anspruch 6, wobei das Polyacrylat ein Copolymer ist, gebildet aus Monomeren, ausgewählt aus Butylmethacrylat, (2-Dimethylaminoethyl)-Methacrylat und Methylmethacrylat.

8. Feste Einheitsdosierungsform nach Anspruch 6 oder 7, wobei die Filmbeschichtung Magnesiumstearat, basisch butyliertes Methacrylat-Copolymer, Dibutylsebacat und Natriumlaurylsulfat umfasst.

9. Feste Einheitsdosierungsform nach einem der vorangehenden Ansprüche, wobei die Filmbeschichtung ein pH-abhängiges Freisetzungsprofil aufweist.

10. Feste Einheitsdosierungsform nach Anspruch 9, wobei die Filmbeschichtung zu einer *in vitro* Auflösung von Aliskiren von ungefähr 30 % oder weniger nach 5 Minuten, ungefähr 80 % oder weniger nach 10 Minuten und ungefähr 95 % oder weniger nach 15 Minuten führt, bei einem pH-Wert von zwischen ungefähr 2 und 4,5, wobei der Test zum Messen der Auflösung des individuellen Wirkstoffs gemäß United States Pharmacopeia US <711> bei einem pH-Wert von 2,0 unter Verwendung eines Korbverfahrens bei 100 Umdrehungen pro Minute ausgeführt wird und das Auflösungsmedium ein Phosphatpuffer ist.

11. Feste Einheitsdosierungsform nach Anspruch 10, wobei die Filmbeschichtung ein basisch butyliertes Methacrylat-Copolymer umfasst.

12. Feste Einheitsdosierungsform nach einem der Ansprüche 1 bis 5, wobei die Filmbeschichtung eine wässrige Ethylcellulose-Dispersion und optional Hydroxypropylmethylcellulose umfasst.

13. Feste Einheitsdosierungsform nach Anspruch 12, wobei die Filmbeschichtung Dibutylsebacat, wässrige Ethylcellulose-Dispersion, Hydroxypropylmethylcellulose und kolloidales Siliciumdioxid umfasst.

14. Feste Einheitsdosierungsform nach einem der Ansprüche 1 bis 5, wobei die Filmbeschichtung ein Ammoniummethacrylat-Copolymer, insbesondere ein Ammoniummethacrylat-Copolymer vom Typ A und/oder ein Ammoniummethacrylat-Copolymer vom Typ B umfasst.

15. Feste Einheitsdosierungsform nach Anspruch 14, wobei die Filmbeschichtung kolloidales Siliciumdioxid, Triethylcitrat, Ammoniummethacrylat-Copolymer vom Typ A und/oder ein Ammoniummethacrylat-Copolymer vom Typ B umfasst.

16. Feste Einheitsdosierungsform nach einem der vorangehenden Ansprüche zur Verwendung bei der Behandlung einer Erkrankung oder Beschwerden in einer pädiatrischen Population.

17. Feste orale Dosierungsform nach einem der vorangehenden Ansprüche zur Verwendung bei der Behandlung von Bluthochdruck, kongestiver Herzinsuffizienz, Angina, Herzinfarkt, Arteriosklerose, diabetischer Nephropathie, diabetischer Kardiomyopathie, Niereninsuffizienz, peripherer Gefäßerkrankung, linksventrikulärer Hypertrophie, kognitiven Störungen, Schlaganfall, Kopfschmerzen und chronischer Herzinsuffizienz.

## Revendications

1. Forme posologique unitaire solide pour une administration orale sous la forme d'un mini-comprimé ayant un noyau et un enrobage externe, dans laquelle
- le noyau comprend une quantité thérapeutiquement efficace d'aliskiren, ou un sel pharmaceutiquement acceptable de celui-ci, et
- l'enrobage externe est sous la forme d'une couche pelliculaire qui comprend une matière de masquage de goût choisie parmi les polyacrylates, et/ou un composant d'enrobage à libération modifiée choisi parmi les dérivés de cellulose et les copolymères acryliques, et les mélanges de ceux-ci,
dans laquelle ledit mini-comprimé a une taille comprise entre 1 mm et 4 mm, et dans laquelle ledit mini-comprimé contient entre environ 2 mg et environ 4 mg d'aliskiren.

2. Forme posologique unitaire solide selon la revendication 1, dans laquelle ledit mini-comprimé a une taille comprise entre 1,5 mm et 3 mm.

3. Forme posologique unitaire solide selon la revendication 1, dans laquelle ledit mini-comprimé a une taille comprise entre 1,5 mm et 2,5 mm.

4. Forme posologique unitaire solide selon la revendication 1, dans laquelle ledit mini-comprimé a une taille de 2 mm.

5. Forme posologique unitaire solide selon la revendication 4, dans laquelle ledit mini-comprimé contient 3,125 mg d'aliskiren.

6. Forme posologique unitaire solide selon l'une quelconque des revendications précédentes, dans laquelle le polyacrylate est choisi parmi
a) un copolymère formé à partir de monomères choisis parmi l'acide méthacrylique, les esters de l'acide méthacrylique, l'acide acrylique et les esters d'acide acrylique ;
b) un copolymère formé à partir de monomères choisis parmi le méthacrylate de butyle, le (2- diméthylaminoéthyle)méthacrylate et le méthacrylate de méthyle ; et
c) un copolymère formé à partir de monomères choisis parmi l'acrylate d'éthyle, le méthacrylate de méthyle et le chlorure de méthacrylate de triméthylammonioéthyle.

7. Forme posologique unitaire solide selon la revendication 6, dans laquelle le polyacrylate est un copolymère formé à partir de monomères choisis parmi le méthacrylate de butyle, le (2-diméthylaminoéthyle)méthacrylate et le méthacrylate de méthyle.

8. Forme posologique unitaire solide selon les revendications 6 ou 7, dans laquelle la couche pelliculaire comprend du stéarate de magnésium, un copolymère basique de méthacrylate butylé, du sébaçate de dibutyle et du lauryl sulfate de sodium.

9. Forme posologique unitaire solide selon l'une quelconque des revendications précédentes, dans laquelle ladite couche pelliculaire a un profil de libération dépendante du pH.

10. Forme posologique unitaire solide selon la revendication 9, dans laquelle ladite couche pelliculaire conduit à une dissolution *in vitro* de l'aliskiren d'environ 30% ou moins après 5 minutes, d'environ 80% ou moins après 10 minutes et d'environ 95% ou moins après 15 minutes, à un pH compris entre environ 2 et 4,5, où le test de mesure de la dissolution de l'ingrédient actif individuel est effectué suivant la pharmacopée USP <711> à un pH de 2,0 à l'aide d'une méthode de panier à 100 tours par minute et le milieu de dissolution est un tampon phosphate.

11. Forme posologique unitaire solide selon la revendication 10, dans laquelle ladite couche pelliculaire comprend un copolymère de méthacrylate butylé basique.

12. Forme posologique unitaire solide selon l'une quelconque des revendications 1 à 5, dans laquelle ladite couche pelliculaire comprend une dispersion aqueuse d'éthylcellulose et, facultativement, de l'hypromellose.

13. Forme posologique unitaire solide selon la revendication 12, dans laquelle la couche pelliculaire comprend du sébaçate de dibutyle, une dispersion aqueuse d'éthylcellulose, de l'hypromellose et du dioxyde de silicium colloïdal.

14. Forme posologique unitaire solide selon l'une quelconque des revendications 1 à 5, dans laquelle ladite couche pelliculaire comprend un copolymère de méthacrylate d'ammonium, en particulier un copolymère de méthacrylate d'ammonium de type A et/ou un copolymère de méthacrylate d'ammonium de type B.

15. Forme posologique unitaire solide selon la revendication 14, dans laquelle la couche pelliculaire comprend du dioxyde de silicium colloïdal, du citrate de triéthyle, un copolymère de méthacrylate d'ammonium de type A et/ou un copolymère de méthacrylate d'ammonium de type B.

16. Forme posologique unitaire solide selon l'une quelconque des revendications précédentes, pour une utilisation dans le traitement d'une maladie ou d'un état dans une population pédiatrique.

17. Forme posologique orale solide selon l'une quelconque des revendications précédentes, pour une utilisation dans le traitement de l'hypertension artérielle, l'insuffisance cardiaque congestive, l'angine de poitrine, l'infarctus du myocarde, l'athérosclérose, la néphropathie diabétique, la myopathie cardiaque diabétique, l'insuffisance rénale, la maladie vasculaire périphérique, l'hypertrophie du ventriculaire gauche, la dysfonction cognitive, l'accident vasculaire cérébral, les maux de tête et l'insuffisance cardiaque chronique.
